# EUROPÄISCHE PATENTSCHRIFT

(11) **EP 3 075 340 B1**
(45) Veröffentlichungstag und Bekanntmachung des Hinweises auf die Patenterteilung: **02.08.2023**
(21) Anmeldenummer: 15161680.2
(22) Anmeldetag: 30.03.2015
(51) Int. Cl.: A61B 18/14

(54) **Gewebeschere für biologisches Gewebe**
TISSUE SHEARS FOR BIOLOGICAL TISSUE
CISEAUX CHIRURGICAUX POUR TISSUS BIOLOGIQUES

(43) Veröffentlichungstag der Anmeldung: 05.10.2016
(73) Patentinhaber: Erbe Elektromedizin GmbH, 72072 Tübingen (DE)
(72) Erfinder: Hiller, Jürgen, 72581 Dettingen (DE)
(74) Vertreter: Rüger Abel Patentanwälte PartGmbB

(56) Entgegenhaltungen:
- EP-A1- 0 517 244
- EP-A1- 2 554 132
- US-A- 5 484 436
- US-A- 6 106 542
- US-B1- 6 312 430
- US-B1- 6 447 511

## Beschreibung

Die Erfindung betrifft eine Gewebeschere zum Durchtrennen von biologischem Gewebe unter gleichzeitiger Versiegelung der erzeugten Geweberänder durch Denaturierung von Proteinen.

Die US 5,925,039 offenbart ein elektrochirurgisches Instrument in Gestalt eines Skalpells oder auch einer Schere, bei dem bzw. der eine aktive Elektrodenfläche aus einem elektrisch leitenden Hartmetall, wie beispielsweise Wolframkarbid besteht. Dieses kann als Schicht auf einen elektrisch leitenden oder auch nichtleitenden Träger aufgebracht sein. Die Hartmetalloberfläche zeigt gegenüber Flächen aus Edelstahl aufgrund ihrer besseren Wärmeleitung eine geringere Gewebeanhaftung.

Die US 2013/0338663 A1 offenbart einen Ablationskatheter, dessen Ablationselektrode in thermischem Kontakt zu einem Kühlkörper steht, der jedoch elektrisch von der Ablationselektrode isoliert ist. Der Kühlkörper kann aus diamantartigem Kohlenstoff (DLC), Aluminiumnitrid, wärmeleitendem Kunstharz, d.h. keramischen oder anderen Nichtleitern bestehen.

Die US 5,925,043 schlägt eine elektrochirurgische Elektrode mit einer leitfähigen Antihaftbeschichtung vor. Dazu ist eine Metallelektrode mit einer Hartstoffschicht (Titannitrid) versehen, die als keramische Schicht angesehen wird. Außerdem werden andere Keramikschichten (Übergangsmetallnitride) vorgeschlagen.

Außerdem offenbart die DE 42 12 063 C1 eine Pinzette, deren Branchen an ihren Enden (von innen nach außen) mit einem Schichtaufbau aus einer Korrosionsschutzschicht, einer Hartstoffschicht und einer Edelmetallschicht versehen sind.

Weiter ist aus der EP 0 517 244 A1 eine Gewebeschere mit zwei aus Edelstahl oder martensitischem Stahl bestehenden Scherenhälften bekannt. Die Scherenhälften sind voneinander elektrisch isoliert und bilden Elektroden zur Stromeinleitung in biologisches Gewebe. Mit ihren aufeinander zu weisenden Flachseiten bewegen sich die Scherenhälften der Schere bei der Ausführung eines Schnitts aneinander vorbei. Wenigstens eine der Flachseiten kann mit einer elektrisch isolierenden Beschichtung versehen sein. An den nach außen zu dem Gewebe gerichteten Flächen können die Scherenhälften mit einer elektrisch und thermisch gut leitenden Kupfer- oder Silberbeschichtung versehen sein. Weiterer relevanter Stand der Technik wird in EP 2 554 132 A1, US 5 484 436 A, US 6 447 511 B1, US 6 106 542 A und US 6 312 430 B1 offenbart.

Es ist Aufgabe der Erfindung, eine Gewebeschere zum Durchtrennen von biologischem Gewebe anzugeben, bei deren Anwendung unter weitgehender Schonung umgebenden Gewebes saubere und gut versiegelte Schnittränder erzeugt werden.

Diese Aufgabe wird mit der Gewebeschere nach Anspruch 1 gelöst.

Die erfindungsgemäße Gewebeschere weist zwei Branchen auf, die an ihren aufeinander zu weisenden Gleitflächen mit unterschiedlichen Schichten versehen sind. Während die erste Branche vorzugsweise an ihrer Scheidefläche mit einer elektrisch leitenden Hartstoffschicht versehen ist, ist die zweite Branche mit einer elektrisch isolierenden keramischen Schicht versehen. Weil eine der beiden Gleitflächen elektrisch nicht leitet, die andere jedoch leitend ist, entsteht eine elektrische Asymmetrie. Durch die Beschichtung der einen Branche mit einer elektrisch nicht leitenden Keramik, entsteht ein weitgehend stromfreies Gebiet in der Nähe der beschichteten Fläche dieser Branche sowie eine Stromkonzentration in der Nähe der zugehörigen Schneidkante. Zusätzlich wird durch die Beschichtung der Energiefluss geführt. Dies bedeutet, beim Trennen von biologischem Gewebe fließt die Energie, d.h. der Strom, von der Schneidfläche der ersten Branche über den Rücken der zweiten Branche zur Neutralelektrode.

Die gewünschte physiologische Wirkung wird unterstützt durch effektive Kühlung der mit der isolierenden keramischen Schicht versehenen Branche. Die Wärmeverteilung und -abfuhr wird hier durch einen Hartmetallkörper erreicht. Der Hartmetallkörper kann z.B. aus Wolframkarbid oder einem geeigneten anderen Sintermetall bestehen. Der vorzugsweise nur an einer der Branchen angebrachte Hartmetallkörper führt zu einer thermischen Asymmetrie. Diese kann dazu genutzt werden den der Keramikschicht innewohnenden thermischen Widerstand zu kompensieren.

Der Hartmetallkörper liegt an der Rückenfläche der Branche frei, so dass der Hartmetallkörper dort die in der Nähe der Schneidkante und an der Keramikschicht aufgenommene Wärme verteilt ableiten kann, ohne die Koagulationsfunktionalität zu beeinträchtigen.

Der Hartmetallkörper steht insbesondere in Nachbarschaft der Schneidkante mit der keramischen Schicht in Kontakt. Vorzugsweise erstreckt sich der Hartmetallkörper über die gesamte Länge der Schneidkante. Er kann sich außerdem, wenigstens an einigen Stellen der Branche, rechtwinklig zur Schneidkante gemessen über die gesamte Breite der keramischen Schicht erstrecken. Vorzugsweise gilt dies entlang der gesamten Schneidkante.

Durch den thermischen Kontakt zwischen der Gleitfläche bzw. der darauf befindlichen keramischen Schicht und dem Hartmetallkörper wird eine Kühlungszone gebildet, in der der geschnittene und koagulierte Rand des biologischen Gewebes gekühlt wird, so dass eine unkontrollierte Fortschreitung der Koagulation vermieden wird. Zusätzlich wird durch die verbesserte Wärmeabfuhr das Anhaften des Gewebes an der Branche reduziert, vorzugsweise vermieden.

Die mit der Hartstoffschicht (z.B. Titannitrid) versehene Branche weist einen Hartmetallkörper auf, um hier das Wärmemanagement zu beeinflussen.

Typischerweise besteht die erste Branche aus Stahl, während die zweite Branche aus einer Kombination des Hartmetallkörpers und eines Trägers aus Stahl besteht. Der Hartmetallkörper und der Träger aus Stahl können über eine Naht miteinander verbunden sein, die als Löt- oder Schweißnaht ausgebildet sein kann. Damit ist eine spaltfreie Verbindung zwischen dem Träger aus Stahl und dem Hartmetallkörper gegeben. Es ist möglich, dass beide Branchen mit einem Hartmetallkörper versehen sind.

Die Hartstoffschicht und/oder die keramische Schicht erstrecken sich vorzugsweise über die gesamte Länge der jeweiligen Branche durch das Gelenk hindurch. Die elektrisch nicht leitende Keramikschicht unterstützt die elektrische Isolierung der Branchen gegeneinander. Außerdem tragen sowohl die Hartstoffschicht als auch die keramische Schicht zur Verschleißarmut der Gelenkverbindung und somit zur präzisen Führung der Branchen wesentlich bei, wodurch auch bei langem Gebrauch und Einsatz und nach vielmaliger Sterilisation immer ein sauberer Schnitt am Gewebe erreicht wird. Durch die Wahl unterschiedlicher Werkstoffe für die Hartstoffschicht und die keramische Schicht lässt sich eine Reibpaarung mit geringer Reibung und hoher Standfestigkeit schaffen. Dadurch kann die Gewebeschere besonders leichtgängig und langlebig ausgebildet werden.

Weitere Einzelheiten vorteilhafter Ausführungsformen der Erfindung ergeben sich aus Ansprüchen, der Zeichnung oder der Beschreibung. Es zeigen:
Fig. 1 die erfindungsgemäße Gewebeschere in einer ersten perspektivischen Ansicht,
Fig. 2 die Gewebeschere nach Fig. 1 in einer zweiten perspektivischen Ansicht sowie ein speisendes Gerät in Prinzipdarstellung,
Fig. 3 eine Branche der Gewebeschere nach Fig. 1 und 2,
Fig. 4 die Gewebeschere nach Fig. 2 in einer perspektivischen ausschnittsweisen Darstellung,
Fig. 5 die Gewebeschere nach Fig. 4 geschnitten entlang der Linie V-V in vereinfachter Darstellung unter Weglassung von Gelenk und Handhebel; unter Darstellung des sich im Einsatz ergebenden Stromflusses,
Fig. 6 die geschnittenen Branchen ähnlich Fig. 4 vor dem Schnitt nebst sich ergebendem Stromfluss und
Fig. 7 die Gewebeschere nach Fig. 4 am Schnittpunkt in einer Darstellung ähnlich Fig. 5 und 6.

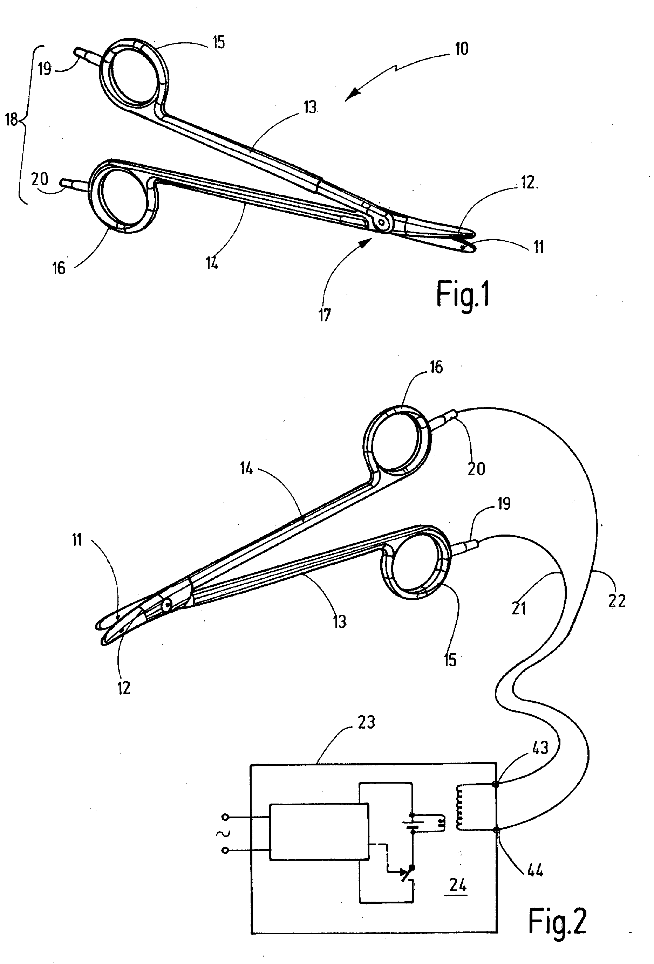

In Fig. 1 ist eine Gewebeschere 10 veranschaulicht, die dazu dient, biologisches Gewebe zu durchtrennen und, falls gewünscht, die dabei entstehenden Gewebesäume durch Koagulation bzw. Kauterisation zu versiegeln. Die Gewebeschere 10 weist eine erste Branche 11 und eine zweite Branche 12 auf, die jeweils in Handhebel 13, 14 übergehen, die an ihren Enden Griffteile 15, 16 oder sonstige Handhabungsmittel aufweisen können. Die Branchen 11, 12 sind an einem Gelenk 17 schwenkbar aneinander gehalten, so dass die Handhebel 13, 14 aufeinander zu und voneinander weg schwenkbar sind.

Die Gewebeschere 10 ist mit einem elektrischen Anschluss 18 versehen, zu dem ein doppelter oder zwei einfache Steckkontaktmittel 19, 20 gehören, der / die an einem Griffteil 15, 16 oder an beiden angeordnet ist / sind. Die Stecckontaktmittel sind vorzugsweise Steckstifte, an die Leitungen 21, 22 anschließbar sind, wie sie aus Fig. 2 schematisch hervorgehen und die mittels eines ersten Pols 43 und eines zweiten Pols 44 zum Anschluss an ein speisendes elektrisches Gerät 23 dienen.

Das elektrische Gerät 23 enthält einen HF-Generator 24, der hochfrequente elektrische Spannung (vorzugsweise zwischen 200 kHz und 4 MHz) abgibt, um damit die Branchen 11, 12 zu speisen, um eine HF-Koagulation des biologischen Gewebes herbeizuführen. Bedienmittel zum gesteuerten Ein- und Ausschalten des Generators 24 sind vorhanden, in Fig. 2 jedoch nicht gesondert veranschaulicht.

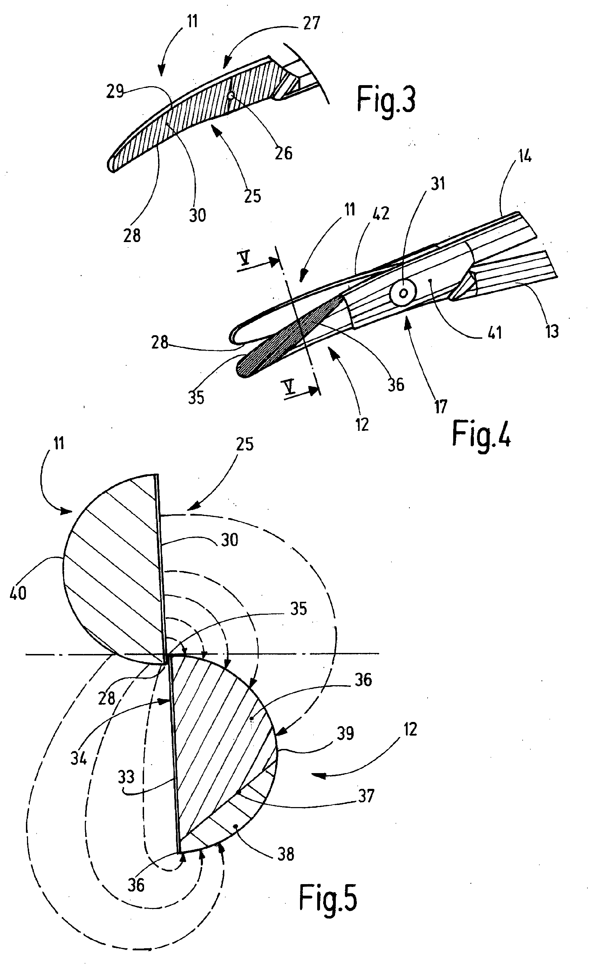

In Fig. 3 ist die erste Branche 11 gesondert veranschaulicht. Sie weist eine Gleitfläche 25 auf, die im Wesentlichen eben ausgebildet ist und sich über den durch eine Bohrung 26 markierten Gelenkbereich 27 hinaus erstreckt. Die Gleitfläche 25 ist vorzugsweise im Wesentlichen eben ausgebildet und erstreckt sich bis zu dem distalen Ende der Branche 11 hin. Die Gleitfläche 25 schließt an eine Schneidkante 28 der Branche 11 an und erstreckt sich bis zur gegenüberliegenden Kante 29. Die Gleitfläche 25 kann, wie dargestellt, unterbrechungsfrei ausgebildet und mit einer Hartstoffschicht 30 versehen sein. Die Hartstoffschicht 30 schließt mindestens an die Schneidkante 28 an und kann sich im Weiteren unterbrechungsfrei über die gesamte Gleitfläche 25 erstrecken. Insbesondere kann sich die Hartstoffschicht 30 bis zu der Kante 29 hin erstrecken. Die Kante 29 ist der Schneidkante 28 gegenüber angeordnet. Außerdem kann die Hartstoffschicht 30 Teile der sonstigen Oberfläche der Branche 11 einnehmen.

Die Hartstoffschicht 30 ist eine elektrisch leitende Schicht, wie beispielsweise eine Titannitridschicht oder eine andere insbesondere elektrisch leitende Hartstoffschicht (Titancarbid, Titancarbonitrid usw.).

Fig. 4 veranschaulicht die Branche 11 in Verbindung mit der Branche 12, die an dem Gelenk 17 durch einen Niet 31, eine Schraube oder dergleichen Verbindungsmittel schwenkbar miteinander verbunden sind. Das Verbindungsmittel ist an seiner Außenseite mit einer Isolation versehen. Dadurch wird ein ungewollter Stromfluss vermieden. Die zweite Branche 12 ist an ihrer der ersten Branche 11 zugewandten Gleitfläche 32 mit einer keramischen Schicht 33 (Fig. 5) versehen, die sich von der Hartstoffschicht 30 chemisch und/oder strukturell unterscheidet. Die keramische Schicht 33 bildet eine Gleitfläche 34, die sich bis zu dem distalen Ende der zweiten Branche 12 erstreckt. Außerdem erstreckt sich die keramische Schicht 33 und somit die Gleitfläche 34 von der Schneidkante 35 der zweiten Branche 12 bis zu der gegenüberliegenden Kante 36, die in Längsrichtung der Branche 12 verläuft. Die vorzugsweise ebene Gleitfläche 34 erstreckt sich vorzugsweise von dem distalen Ende der Branche 12 bis zu dem Gelenk 17 und darüber hinaus. Vorzugsweise ist die keramische Schicht 33 vollflächig unterbrechungsfrei ausgebildet, so dass die ansonsten aus Metall bestehende Branche 12 keinen elektrischen Kontakt zu der Branche 11 hat; mit anderen Worten, von dieser elektrisch isoliert ist. Zu diesem Zweck kann ergänzend der Niet 31 aus Kunststoff, aus Keramik oder aus mit Isolationsmaterial umhülltem Metall ausgebildet sein.

Wie Fig. 5 andeutet, besteht die Branche 11 aus einem einheitlichen Material, z.B. Stahl, der an der Gleitfläche 25 die Hartstoffschicht 30 trägt. Demgegenüber weist die Branche 12 einen Hartmetallkörper 36 auf, der sich über einen erheblichen Teil entlang der Länge der zweiten Branche 12 erstreckt (siehe Fig. 4). Vorzugsweise erstreckt sich der Hartmetallkörper 36 an dem distalen Ende der Branche 12 von der Schneidkante 35 bis zu der unteren Kante 36. In Richtung zu dem Gelenk 17 kann der Querschnitt des Hartmetallkörpers 36, der gesondert aus Fig. 5 hervorgeht, flächenmäßig abnehmen. Die Länge des Hartmetallkörpers 36 ist vorzugsweise größer als die Hälfte des Abstandes des distalen Endes der Branche 12 von dem Gelenk 17. Der Hartmetallkörper 36 kann, wie Fig. 5 zeigt, an einer Naht 37 mit einer Trägerstruktur 38 der Branche 12 verbunden sein. Die Trägerstruktur 38 besteht z.B. aus Stahl und verjüngt sich ausgehend von dem vollen Querschnitt der Branche 12 zu dem distalen Ende hin auf Null. Die Trägerstruktur 38 endet vor Erreichen des distalen Endes. Die Naht 37 kann eine Lötnaht, Schweißnaht oder dergleichen stoffliche Verbindung sein. An der von der Gleitfläche 34 abgewandten Rückseite 39 der Branche 12 liegen sowohl der Hartmetallkörper 36 als auch die Trägerstruktur 38 frei zu Tage. Entsprechendes gilt für die Branche 11. Auch dort ist die von der Gleitfläche 25 abgewandte Rückseite 40 unbedeckt.

Wie Fig. 4 erkennen lässt, sind die Handhebel 13, 14 mit einer elektrisch isolierenden Schicht 41, 42 versehen, die sich vorzugsweise bis zu den Branchen 11, 12 erstreckt, diese aber an den Gleitflächen 25, 34 sowie Bereiche an der Rückseiten 39, 40 frei lässt. Die Abdeckung der Branchen 11, 12 durch die isolierende Schicht 41, 42 kann abhängig vom Verwendungszweck gebildet sein. Es ist möglich, dass nahezu die komplette Rückseite 39, 40 durch die isolierende Schicht 41, 42 abgedeckt ist und die Branchen nur im Bereich des distalen Endes ohne isolierende Schicht ausgebildet sind.

Die insoweit beschriebene Gewebeschere 10 arbeitet wie folgt:

In den Fig. 5, 6 und 7 sind die Branchen 11, 12, geschnitten entlang der in Fig. 4 eingetragenen Schnittlinie V-VII, in verschiedenen Schließstadien veranschaulicht.

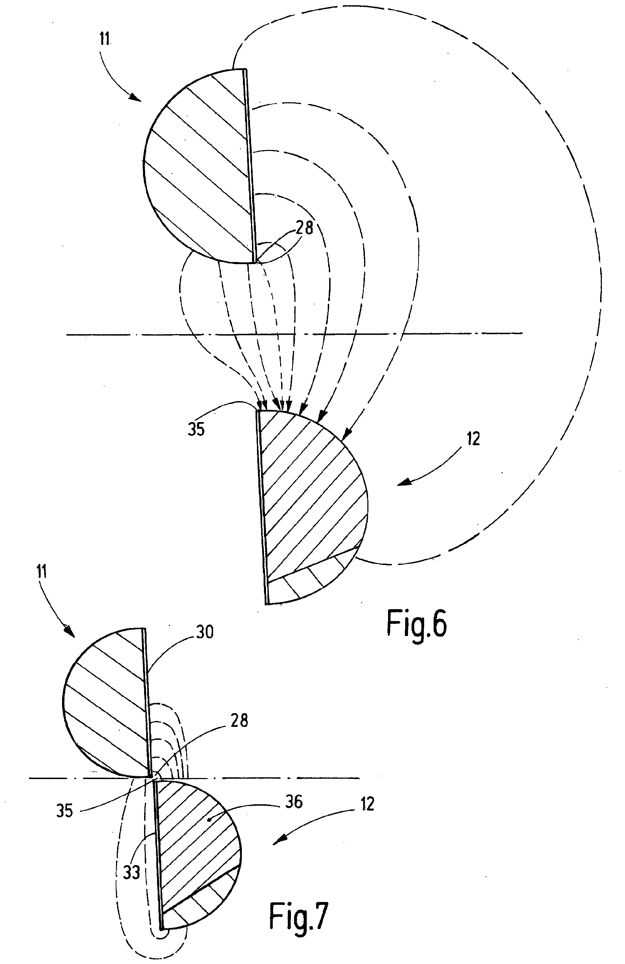

Fig. 5 zeigt die Branchen 11, 12, nachdem sie nicht weiter veranschaulichtes biologisches Gewebe gefasst haben und dabei sind, dieses vor dem Durchtrennen mechanisch unter Druck zu setzen. Wenn nun gleichzeitig der Generator 24 aktiviert wird, erzeugt dieser eine hochfrequente elektrische Durchströmung des Gewebes, deren Stromlinien in Fig. 6 gestrichelt angedeutet sind. Die Stromdichte nimmt mit dem Fortschreiten des Schnittes zu, wie Fig. 7 veranschaulicht. Durch die elektrisch isolierende Wirkung der keramischen Schicht 33 wird ein elektrischer Kurschluss an den Schneidkanten 28, 35 vermieden. In unmittelbarer Nähe der Schneidkanten 28, 35 ergibt sich jedoch eine hohe Stromdichte, die das Gewebe erhitzt und koaguliert. Die in der entstehenden Wärme denaturierenden Eiweiße haben Klebepotential. Würden diese an den Branchen 11, 12 anhaften oder festkleben, würde dies zu einer Behinderung der Arbeit und im schlimmsten Fall zur Unterbrechung des Stromflusses führen. In der Folge wäre die Koagulationswirkung der Gewebeschere nicht mehr vorhanden oder sehr stark eingeschränkt, was wiederum zu einem qualitativ schlechtem Ergebnis führen würde. Eine durch denaturiertes Eiweiß verschmutzte Branche beeinflusst die Koagulationsfunktion der Gewebeschere massiv. Da der Anwender diese Beeinflussung möglicherweise nicht sofort erkennt, kann es dazu führen, dass er weiterhin Gewebe trennt, (die Schneidefunktion wird nicht beeinflusst) ohne dabei die Gefäße erfolgreich zu versiegeln. Im Ergebnis kann es deshalb zu Blutungen kommen. Der Hartmetallkörper 36, dessen Wärmeleitfähigkeit höher ist als die Wärmeleitfähigkeit von Stahl, wirkt dem Anhaften von Gewebe entgegen. Der Hartmetallkörper 36 bewirkt eine wirksame Kühlung der aus Stahl hergestellten Branche 12 und deren keramischen Schicht 33. Dies ergibt in Verbindung mit der haftungsmindernden Hartstoffschicht 30, deren Kühlung durch diese Schicht selbst, sowie gegebenenfalls durch die Branche erfolgt, ein verbessertes Schneide- bzw. Koagulationsergebnis. Insbesondere können die Verhältnisse so eingestellt werden, dass die Kühlwirkung der Hartstoffschicht 30 in Verbindung mit der aus Stahl ausgebildeten Branche 11 vergleichbar ist mit der Kühlwirkung der Kombination aus der keramischen Schicht 33 und dem Hartmetallkörper 36. Die geringe Wärmeleitfähigkeit der keramischen Schicht 33 wird durch die hohe Wärmeleitfähigkeit des Hartmetallkörpers 36 ausgeglichen.

Der fortgesetzte Schnitt führt zu einem Bild der elektrischen Durchströmung wie in Fig. 5 angedeutet. Obwohl die Branchen 11, 12 strukturell und elektrisch asymmetrisch ausgebildet sind, ergibt sich an den schneidkantennahen Bereichen der Rückseiten 39, 40 eine vergleichbare elektrische Durchströmung und somit eine vergleichbare Koagulationswirkung.

Eine Gewebeschere 10 mit verbesserter Standfestigkeit und verbesserter Handhabung weist zwei baulich und elektrisch unterschiedliche Branchen 11, 12 auf. Während die erste Branche 11 seitens ihrer Gleitfläche 25 mit einer metallkeramischen Hartstoffschicht 30, wie beispielsweise Titannitrid versehen ist, ist die zweite Gleitfläche 34 der zweiten Branche 12 mit einer elektrisch nicht leitenden keramischen Schicht 33 versehen. Die Werkstoffpaarung ergibt eine große mechanische Abriebfestigkeit im Lager 17 und an den Schneidkanten 28, 35. Mindestens eine der Branchen, insbesondere die Branche 12 kann zur verbesserten Kühlung und/oder zur Scharfhaltung der Schneidkante 35 (28) mit einem Hartmetallkörper 36 versehen sein, um ein Erhitzen der Branchen 11, 12 während der Koagulation und ein Ankleben des Gewebes zu verhindern.

### Bezugszeichen:

| | |
|---|---|
| 10 | Gewebeschere |
| 11 | erste Branche |
| 12 | zweite Branche |
| 13, 14 | Handhebel |
| 15, 16 | Griffteil |
| 17 | Gelenk |
| 18 | elektrischer Anschluss |
| 19, 20 | Steckkontaktmittel |
| 21, 22 | Leitungen |
| 23 | Gerät |
| 24 | Generator |
| 25 | erste Gleitfläche |
| 26 | Bohrung |
| 27 | Gelenkbereich |
| 28 | Schneidkante der ersten Branche 11 |
| 29 | der Schneidkante 28 gegenüberliegende Kante |
| 30 | Hartstoffschicht |
| 31 | Niet |
| 32 | zweite Gleitfläche |
| 33 | keramische Schicht |
| 34 | zweite Gleitfläche |
| 35 | Schneidkante der zweiten Branche 12 |
| 36 | Hartmetallkörper |
| 37 | Naht |
| 38 | Trägerstruktur |
| 39, 40 | Rückseiten |
| 41, 42 | Isolierende Schicht |
| 43, 44 | Erster Pol, zweiter Pol |
| | |

## Patentansprüche

1. Gewebeschere (10) zum Durchtrennen von biologischem Gewebe
mit einer ersten Branche (11), die eine erste Gleitfläche (25) und eine von der Gleitfläche (25) abgewandte Rückseite (40) aufweist, die an einer ersten Schneidkante (28) endet,
mit einer zweiten, eine Trägerstruktur (38) aufweisenden Branche (12), mit der ein Hartmetallkörper (36) verbunden ist und die eine Rückseite (39) und eine zweite Gleitfläche (34) aufweist, die an einer zweiten Schneidkante (35) endet, wobei an der zweiten Gleitfläche (34) eine keramische Schicht (33) vorgesehen ist, wobei der Träger (38) aus Stahl besteht und der Hartmetallkörper (36) eine höhere Wärmeleitfähigkeit als Stahl besitzt,
mit einem Gelenk (17), an dem die beiden Branchen (11, 12) schwenkbar miteinander verbunden sind, so dass die Gleitflächen (25, 34) aufeinander zu weisen und die erste Schneidkante (28) an der zweiten Schneidkante (35) vorbei bewegbar ist,
wobei an der ersten Gleitfläche (28) eine elektrisch leitende Hartstoffschicht (30) angeordnet und die an der zweiten Gleitfläche (34) vorgesehene keramische Schicht (33) von der Hartstoffschicht (30) der ersten Gleitfläche (28) verschieden ist und dass der Hartmetallkörper (36) mit der Gleitfläche (34) in wärmeaustauschendem Kontakt steht und an der von der Gleitfläche (34) weg weisenden Rückseite (39) der Branche (12) frei liegt so dass an der Rückseite (39) der Branche (12) sowohl der Hartmetallkörper (36) als auch die Trägerstruktur (38) frei liegen und die Rückseite (40) unbedeckt ist.

2. Gewebeschere nach einem der vorstehenden Ansprüche, wobei der Hartmetallkörper (36) sich über die gesamte Länge der Schneidkante (35) erstreckt.

3. Gewebeschere nach Anspruch 1 und 2, wobei der Hartmetallkörper (36) und der Träger (38) über eine Naht (37) miteinander verbunden sind.

4. Gewebeschere nach Anspruch 3, wobei die Naht (37) eine Löt- oder Schweißnaht ist.

5. Gewebeschere nach Anspruch 3 oder 4, wobei sich die Naht (37) in einer Richtung von dem Gelenk (17) weg erstreckt.

6. Gewebeschere nach einem der vorstehenden Ansprüche, wobei die Keramikschicht (33) die Naht (37) bedeckend ausgebildet ist.

7. Gewebeschere nach einem der vorstehenden Ansprüche, wobei sich die erste Gleitfläche (25) und die Hartstoffschicht (33) durch das Gelenk (17) hindurch erstrecken.

8. Gewebeschere nach einem der vorstehenden Ansprüche, wobei sich die zweite Gleitfläche (34) und die Keramikschicht (33) durch das Gelenk (17) hindurch erstrecken.

9. Gewebeschere nach einem der vorstehenden Ansprüche, wobei die erste Branche (11) und die zweite Branche (12) jeweils mit einem Handhebel (13, 14) verbunden sind.

10. Gewebeschere nach einem der vorstehenden Ansprüche, wobei die Handhebel (13, 14) mit einer elektrischen Isolierschicht (41, 42) versehen sind.

11. Gewebeschere nach einem der vorstehenden Ansprüche, wobei die erste Branche (11) mit einem ersten Pol (43) eines elektrischen Geräts (24) und die zweite Branche (12) mit einem zweiten Pol (44) des elektrischen Geräts (24) verbindbar ausgebildet ist.

## Claims

1. Tissue shears (10) to cut biological tissue comprising:
a first branch (11) having a first sliding surface (25) and a back (40) facing away from the sliding surface (25), the back (40) terminating at a first cutting edge (28),
a second branch (12) having a support layer (38) and being connected to a cermet body (36) and having a back (39) and a second sliding surface (34) that terminates at a second cutting edge (35), wherein a ceramic layer (33) is provided on the second sliding layer (34), wherein the support (38) consists of steel, and the cermet body (36) has a higher thermal conductivity than steel;
a joint (17) at which the two branches (11, 12) are pivotably connected with one another, so that the sliding surfaces (25, 34) face one another and the first cutting edge (28) can be moved past the second cutting edge (35);
wherein an electrically conductive hard material layer (30) is arranged on the first sliding surface (28) and the ceramic layer (33) arranged on the second sliding surface (34) differs from the hard material layer (30) of the first sliding surface (28) and that the cermet body (36) is in heat exchange contact with the sliding surface (34) and is exposed on the back (39), facing away from the sliding surface (34), of the branch (12) so that the cermet body (36) as well as the support structure (38) are exposed on the back (39) of the branch (12) and the back (40) is covered.

2. The tissue shears described in the preceding claim, wherein the cermet body (36) extends over the entire length of the cutting edge (35).

3. The tissue shears described in claim 1 and 2 wherein the cermet body (36) and the support (38) are connected with one another through a seam.

4. The tissue shears described in claims 3 wherein the seam (37) is a soldered seam or a weld seam.

5. The tissue shears described in one of claims 3 and 4 wherein the seam (37) extends in a direction away from the joint (17).

6. The tissue shears described in one of the preceding claims wherein the ceramic layer (33) is designed to cover the seam (37).

7. The tissue shears described in one of the preceding claims wherein the first sliding surface (25) and the hard material layer (33) extend through the joint (17).

8. The tissue shears described in one of the preceding claims wherein the second sliding surface (34) and the ceramic layer (33) extend through the joint (17).

9. The tissue shears described in one of the preceding claims, **characterized in that** the first branch (11) and the second branch (12) are each connected with a handle (13, 14).

10. The tissue shears described in one of the preceding claims wherein the handle (13, 14) has a layer of electrical insulation (41, 42).

11. The tissue shears described in one of the preceding claims wherein the first branch (11) is designed to allow connection with a first terminal (43) of an electrical device (24) and the second branch (12) is designed to allow connection with a second terminal (44) of the electrical device (24).

## Revendications

1. Ciseaux à tissus (10) destinés à couper des tissus biologiques,
comprenant un premier mors (11) qui présente une première surface de glissement (25) et une face arrière (40) qui est détournée de la surface de glissement (25) et se termine sur une première arête de coupe (28),
comprenant un deuxième mors (12) présentant une structure de support (38) à laquelle est relié un corps en métal dur (36) et qui présente une face arrière (39) et une deuxième surface de glissement (34), qui se termine sur une deuxième arête de coupe (35), où une couche céramique (33) est prévue sur la deuxième surface de glissement (34), où le support (38) est en acier et le corps en métal dur (36) présente une conductivité thermique plus élevée que l'acier,
comprenant une articulation (17) sur laquelle les deux mors (11, 12) sont reliés l'un à l'autre de façon pivotante, de sorte que les surfaces de glissement (25, 34) sont tournées l'une vers l'autre et la première arête de coupe (28) peut être déplacée en passant devant la deuxième arête de coupe (35),
sachant qu'une couche de matériau dur (30) électriquement conductrice est disposée sur la première surface de glissement (28) et la couche céramique (33) prévue sur la deuxième surface de glissement (34) est différente de la couche de matériau dur (30) de la première surface de glissement (28), et que le corps en métal dur (36) est en contact d'échange de chaleur avec la surface de glissement (34) et est dégagé sur la face arrière (39) du mors (12) détournée de la surface de glissement (34), de sorte que sur la face arrière (39) du mors (12), aussi bien le corps en métal dur (36) que la structure de support (38) sont dégagés et la face arrière (40) est découverte.

2. Ciseaux à tissus selon l'une des revendications précédentes, dans lesquels le corps en métal dur (36) s'étend sur toute la longueur de l'arête de coupe (35).

3. Ciseaux à tissus selon les revendications 1 et 2, dans lesquels le corps en métal dur (36) et le support (38) sont reliés l'un à l'autre par un joint (37).

4. Ciseaux à tissus selon la revendication 3, dans lesquels le joint (37) est un joint brasé ou soudé.

5. Ciseaux à tissus selon la revendication 3 ou 4, dans lesquels le joint (37) s'étend dans une direction s'éloignant de l'articulation (17).

6. Ciseaux à tissus selon l'une des revendications précédentes, dans lesquels la couche céramique (33) est réalisée de manière à recouvrir le joint (37).

7. Ciseaux à tissus selon l'une des revendications précédentes, dans lesquels la première surface de glissement (25) et la couche céramique (33) s'étendent à travers l'articulation (17).

8. Ciseaux à tissus selon l'une des revendications précédentes, dans lesquels la deuxième surface de glissement (34) et la couche céramique (33) s'étendent à travers l'articulation (17).

9. Ciseaux à tissus selon l'une des revendications précédentes, dans lesquels le premier mors (11) et le deuxième mors (12) sont reliés respectivement à un levier à main (13, 14).

10. Ciseaux à tissus selon l'une des revendications précédentes, dans lesquels les leviers à main (13, 14) sont pourvus d'une couche d'isolation électrique (41, 42).

11. Ciseaux à tissus selon l'une des revendications précédentes, dans lesquels le premier mors (11) est réalisé de manière à pouvoir être relié à un premier pôle (43) d'un appareil électrique (24) et le deuxième mors (12) est réalisé de manière à pouvoir être relié à un deuxième pôle (44) de l'appareil électrique (24).
